# EUROPÄISCHE PATENTSCHRIFT

(11) **EP 3 129 689 B1**
(45) Veröffentlichungstag und Bekanntmachung des Hinweises auf die Patenterteilung: **14.07.2021**
(21) Anmeldenummer: 15711516.3
(22) Anmeldetag: 23.03.2015
(51) Int. Cl.: A61M 39/22, F16K 41/10, F16K 31/06, F02M 63/00

(54) **ELEKTROMAGNETISCH BETÄTIGTES VENTIL**
ELECTROMAGNETICALLY ACTUATED VALVE
SOUPAPE ÉLECTROMAGNÉTIQUE

(30) Priorität: 08.04.2014 DE 102014005137
(43) Veröffentlichungstag der Anmeldung: 15.02.2017
(73) Patentinhaber: Fresenius Medical Care Deutschland GmbH, 61352 Bad Homburg (DE); Rausch und Pausch GmbH, 95100 Selb (DE)
(72) Erfinder: SCHIEWECK, Werner, 95199 Thierstein (DE); DÖHLA, Werner, 95482 Gefrees (DE); SEEWALD, Olaf, 95448 Bayreuth (DE); TEICHMANN, Michael, 95444 Bayreuth (DE)
(74) Vertreter: Breuninger, Marcus
(86) Internationale Anmeldenummer: PCT/EP2015/056082
(87) Internationale Veröffentlichungsnummer: WO 2015/154974

(56) Entgegenhaltungen:
- WO-A1-97/15771
- DE-A1- 2 303 450
- DE-A1-102011 077 069
- DE-A1-102012 019 388
- US-A1- 2005 173 664

## Beschreibung

### Technisches Gebiet

Die Erfindung betrifft ein elektromagnetisch betätigtes Ventil, insbesondere zur Verwendung in einer Vorrichtung zum Bereitstellen eines extrakorporalen Kreislaufs zur Behandlung eines Patienten, insbesondere zur Dialyse.

### Darstellung der Erfindung

In Vorrichtungen zum Bereitstellen eines extrakorporalen Kreislaufs zur Behandlung eines Patienten, insbesondere zur Dialyse, werden elektromagnetisch betätigte Ventile eingesetzt, um den Fluidfluss in einem Fluidkanal der Vorrichtung zu steuern. Bei Ventilen in medizinischen Anwendungen ist es wichtig, dass die beweglichen Teile des Ventils nicht mit dem Fluid in Kontakt kommen. Bei dem Fluid kann es sich beispielsweise um Dialyseflüssigkeit (Dialysat) handeln.

Zu diesem Zweck weisen solche Ventile üblicherweise ein Dichtelement auf, welches dazu dient, den Antrieb des Ventils von dem Fluidkanal des Ventils fluiddicht zu trennen. Das Dichtelement weist dazu beispielsweise einen Faltenbalg auf, um eine axiale Beweglichkeit des Dichtelements zu ermöglichen. Dokument WO9715771 beschreibt ein Dichtelement entsprechend der Präambel von Anspruch 1 und 2.

Das Ventil weist des Weiteren einen Ventilschieber mit einem Magnetanker auf, der durch Bestromen einer Spule des Ventils in axialer Richtung entgegen einer Federkraft verlagerbar ist, um den Ventilschieber in eine Schließposition oder aus der Schließposition zu bewegen, in der ein Dichtsitz des Ventils mittels des Dichtelements verschlossen ist. Üblicherweise ist der Magnetanker in einem nicht magnetisierbaren Führungsrohr im Innem der Spule gelagert. Diese Lagerung verursacht Reibungskräfte, die von der Magnetkraft überwunden werden müssen und die den Verschleiß erhöhen. Zusätzlich entstehen durch das nicht-magnetische Führungsrohr magnetische Verluste in dem Eisenkreis des Magnetantriebs des Ventils.

Aufgabe der vorliegenden Erfindung ist es daher, ein elektromagnetisch betätigtes Ventil, insbesondere zur Verwendung in einer Vorrichtung zum Bereitstellen eines extrakorporalen Kreislaufs zur Behandlung eines Patienten, insbesondere zur Dialyse, vorzuschlagen, das die eingangs genannten Nachteile überwindet, insbesondere Verluste und Verschleiß durch Reibung sowie magnetische Verluste reduziert.

Die Aufgabe wird durch ein elektromagnetisch betätigtes Ventil gemäß den Merkmalen der unabhängigen Ansprüche 1 und 2 gelöst. Weiterbildungen und vorteilhafte Ausgestaltungen sind in den abhängigen Ansprüchen angegeben.

Erfindungsgemäß ist der Ventilschieber auf einer dem Dichtsitz zugewandten Seite des Magnetankers mittels mindestens eines Federelements in radialer Richtung ausgerichtet und in axialer Richtung beweglich gelagert. Des Weiteren weist der Ventilschieber einen in axialer Richtung vom Magnetanker abstehenden Führungsstift auf. Der Führungsstift ist vorzugsweise an einem dem Dichtsitz des Ventils gegenüberliegenden axialen Ende des Ventilschiebers angeordnet und besitzt eine Oberfläche mit einem gegenüber dem Außendurchmesser des Magnetankers reduzierten Durchmesser, wobei der Ventilschieber auf dieser Oberfläche des Führungsstifts in axialer Richtung gleitend gelagert ist. Es kann auch vorgesehen sein, den Führungsstift an dem Dichtsitz zugewandten Ende des Ventilschiebers anzuordnen und das Federelement auf einer dem Dichtsitz abgewandten Seite des Magnetankers.

In einer Ausführungsform, die nicht Teil der Erfindung ist, ist auch eine Lagerung des Ventilschiebers ohne Federelement mit jeweils einem Führungsstift als Gleitlager auf beiden axialen Seiten des Ventilschiebers möglich.

Alternativ, aber nicht als Teil der Erfindung, kann die Lagerung auch ohne Gleitlager mittels jeweils eines Federelements auf beiden axialen Seiten des Magnetankers realisiert werden.

In jedem Falle ist der Magnetanker dadurch derart gelagert und ausgerichtet, dass er zumindest im Bereich der Spule berührungsfrei geführt werden kann. Die Lagerung und Ausrichtung des Magnetankers als Teil des Ventilschiebers erfolgt vielmehr auf den beiden axialen Seiten des Magnetankers. Daher kann auch auf ein Führungsrohr zur Lagerung und Führung des Magnetankers selbst verzichtet werden, so dass Reibungsverluste und magnetische Verluste reduziert werden können und letztlich eine hohe Lebensdauer des Ventils erreicht wird. Das Federelement erfüllt außer der Ausübung einer Federkraft als weitere Funktion die reibungsfreie Lagerung. Da diese Funktionen mit einem einzigen Element erzielt werden können, ist das Ventil gemäß der Erfindung bei geringer Bauteilanzahl besonders einfach und robust aufgebaut.

Vorzugsweise ist das Federelement mit dem Ventilschieber und dem Gehäuse oder Gehäuseteilen des Ventils ortsfest verbunden, wobei weiter vorzugsweise das Federelement im entspannten Zustand im Wesentlichen eben ist und sich in einer Ebene senkrecht zur Verschiebeachse des Ventilschiebers erstreckt. Das Federelement kann beispielsweise als werkzeugfallendes Stanzteil aus einem blechförmigen Material gebildet sein. Bevorzugt ist das Federelement im stromlosen Zustand des Ventils jedoch ausgelenkt, beispielsweise um 0,3 mm entlang der Verschiebeachse. Die Auslenkung des an sich ebenen Federelements kann insbesondere aufgrund der auf den Magnetanker wirkenden mechanischen Belastung in Richtung des Dichtsitzes erfolgen, die beispielsweise durch eine Vorspannfeder erzeugt wird. Bei Bestromung der Spule und einer entsprechenden Verlagerung des Ventilschiebers entgegen der mechanischen Belastung wird das Federelement insbesondere aufgrund der ortsfesten Verbindung mit dem Ventilschieber dann in die entgegengesetzte Richtung bezogen auf den ebenen Zustand des Federelements ausgelenkt, vorzugsweise um etwa den gleichen Betrag. Durch die Auslenkung des Federelements um seine Nulllage herum, d.h. den im Wesentlichen ebenen Zustand, wird eine höhere Dauerfestigkeit erreicht, als wenn das Federelement nur in eine Richtung ausgelenkt werden würde.

Bei dem Federelement kann es sich um ein ringförmiges Element mit geschwungenen oder im Wesentlichen S-förmigen Federbeinen handeln, so dass der Ventilschieber mittels des Federelements zentriert wird. Ein solches Federelement erlaubt zudem eine axiale Beweglichkeit, d.h. eine Auslenkung des Ventilschiebers senkrecht zur Ebene des Federelements. Es ist jedoch auch denkbar, den Ventilschieber nicht ortsfest an dem Federelement zu befestigen, so dass nur eine radiale Ausrichtung des Ventilschiebers durch das Federelement erfolgt, der Ventilschieber aber in axialer Richtung relativ zu dem Federelement verlagerbar ist. Vorteilhaft ist das faltenbalgartige Dichtelement an einem Ende geschlossen.

Beispielsweise kann es eine Kappen- oder Hutform besitzen. Das Dichtelement ist vorzugsweise an dem axialen Ende des Ventilschiebers, welches dem Dichtsitz zugewandt ist, derart angeordnet, dass das geschlossene Ende des Dichtelements in der Schließposition des Ventilschiebers den Dichtsitz fluiddicht verschließt. Das Dichtelement ist vorzugsweise als ein integrales Bauteil ausgebildet. Mit anderen Worten, das geschlossene Ende und der Faltenbalg bilden ein Bauteil und sind nicht separat ausgebildet. Bevorzugt besteht das Dichtelement aus einem elastischen Gummimaterial. Das einstückige Dichtelement mit Faltenbalg ist einfacher und kostengünstiger in der Herstellung als ein herkömmliches Dichtelement, welches aus mehreren Einzelteilen hergestellt ist, insbesondere einem Faltenbalg aus PTFE, einer separaten Dichtkappe und einem O-Ring.

Insbesondere ist ein Faltenbalg aus PTFE aufwändig und teuer in der Herstellung, da er beispielsweise durch spanende Verfahren hergestellt wird. Unter einem Faltenbalg wird ganz allgemein jedes schlauchförmig oder ähnlich geformte Element verstanden, welches in seiner axialen Richtung durch Bereitstellen *von* zumindest einer Faltung deformierbar ist. Der Faltenbalg kann beispielsweise nur eine Faltung aufweisen oder auch mehrere Faltungen und somit ziehharmonikaartig geformt sein. In einer vorteilhaften Ausgestaltung umfasst der Ventilschieber einen Stößel, welcher mit dem Magnetanker auf der dem Dichtsitz zugewandten Seite des Magnetankers verbunden ist und das dem Dichtsitz zugewandte axiale Ende des Ventilschiebers bildet. Das Dichtelement ist dabei vorzugsweise auf dem Stößel derart befestigt, dass es sich bei einer Verlagerung des Ventilschiebers mitbewegt Beispielsweise kann das Dichtelement mit seinem Faltenbalg über den Stößel gestülpt oder sogar fest auf den Stößel aufgesteckt sein. Das Dichtelement kann auch auf den Stößel aufvulkanisiert sein. Der Stößel kann an seinem freien axialen Ende einen Vorsprung, beispielsweise eine umlaufende Wulst, aufweisen, über den das elastische Dichtelement gestülpt und somit gehalten werden kann. Entsprechend kann das Dichtelement eine Ausnehmung, beispielsweise eine umlaufende Kerbe, auf seiner Innenseite aufweisen, welche mit dem Vorsprung auf dem Stößel zusammenwirkt.

Vorzugsweise umfasst das Ventil einen Anschlag, welcher mit dem Führungsstift so zusammenwirkt, dass er eine axiale Bewegung des Ventilschiebers begrenzt. Der Anschlag kann ein Dämpfungselement aus einem Gummimaterial umfassen, so dass die Bewegung des Ventilschiebers auf eine dämpfende Weise begrenzt wird, wenn die Stirnfläche des Führungsstifts auf den Anschlag trifft. Insbesondere erfolgt dadurch auch eine Geräuschdämpfung. Der Anschlag kann beispielsweise als Gummischeibe ausgebildet sein, die einfach und kostengünstig herzustellen ist.

Vorteilhaft kann eine Kunststoffummantelung vorgesehen sein, welche zumindest einen Antriebsteil des Ventils umgibt. Die Kunststoffummantelung kann das Ventil vor Spritzwasser und/oder Verschmutzung schützen, beispielsweise gemäß Schutzart IP54, und dient gleichzeitig als thermischer Berührschutz. Die Kunststoffummantelung kann im Niederdruckspritzguss hergestellt sein, wobei gleichzeitig eine Verklebung mit dem Ventilgehäuse und Abdichtung desselben realisiert wird. Dies ist weniger aufwändig und kostengünstiger als eine Pulverbeschichtung oder eine Umspritzung der Spule im Kunststoffspritzguss. Auch eine Ummantelung durch eine Vergussmasse wäre aufwändiger und teurer und benötigte gegebenenfalls zusätzliche Dichtelemente und Verklebungen. Das Ventil kann insbesondere in einer Vorrichtung zum Bereitstellen eines extrakorporalen Kreislaufs zur Behandlung eines Patienten, insbesondere zur Dialyse, vorgesehen sein und verwendet werden, wobei die Vorrichtung zumindest einen Fluidkanal und zumindest ein solches Ventil in dem Fluidkanal zur Steuerung eines Fluidflusses durch den Fluidkanal umfasst. Bei der Vorrichtung kann es sich um ein Dialysegerät beispielsweise zur Hämodialyse handeln.

### Detaillierte Beschreibung der Zeichnungen

Im Folgenden wird ein bevorzugtes Ausführungsbeispiel der Erfindung beispielhaft anhand der begleitenden Zeichnungen beschrieben. Darin zeigen:
Fig. 1 ein erfindungsgemäßes Ventil in einer Schnittdarstellung,
Fig.2 ein Federelement aus dem Ventil aus Fig. 1 und
Fig. 3 schematisch eine Dialysevorrichtung mit dem Ventil aus Fig. 1.

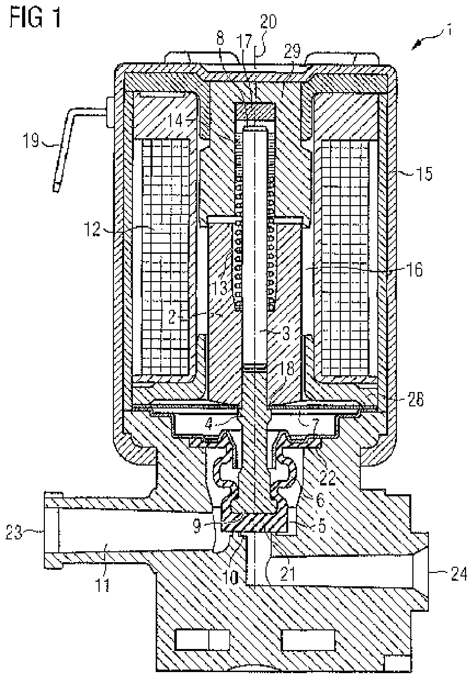

In Fig. 1 ist ein elektromagnetisch betätigtes Ventil 1 dargestellt, welches insbesondere als Dialyseventil Verwendung finden kann. Das Ventil 1 ist zur Steuerung eines Fluidflusses in einem Fluidkanal beispielsweise eines Dialysegeräts vorgesehen. Dazu kann ein Dichtsitz 10 in einem Fluidkanal 11 zwischen Ein- und Austrittsenden 23, 24 des Ventils 1 verschlossen und geöffnet werden. Das Ventil 1 weist einen entlang einer Verschiebeachse 20 axial verschieblichen Ventilschieber auf, welcher einen Magnetanker 2, einen Führungsstift 3 und einen Stößel 4 umfasst, welche mittels Presssitz verbunden sind. Der Magnetanker 2 bildet einen Teil des sogenannten Eisenkreises des Ventils 1 und wird mittels der von einer Spule 12 erzeugt Magnetkraft in axialer Richtung bewegt. In diesem Ausführungsbeispiel wird der Magnetanker 2 entgegen der Kraft einer Feder 13 aus der Schließposition heraus in Richtung eines ortsfesten Polteils 29 bewegt. Die Magnetkraft wirkt axial auch gegen die Kraft eines Federelements 7, das zur Lagerung und Ausrichtung des Ventilschiebers vorgesehen ist, wie unten genauer erläutert. Wenn die Spule 12, wie hier dargestellt, abgeschaltet ist, wird der Ventilschieber in Richtung des Dichtsitzes 10 gedrückt und verschließt diesen mit seinem dem Dichtsitz 10 zugewandten axialen Ende 9.

Zwischen dem Ventilschieber und dem Dichtsitz 10 ist ein Dichtelement 5 angeordnet. Genauer gesagt sitzt das Dichtelement 5 auf dem freien axialen Ende des Stößels 4, welches zugleich das dem Dichtsitz 10 zugewandte axiale Ende 9 des Ventilschiebers bildet. Das freie Ende des Stößels 4 hat einen Vorsprung, beispielsweise eine umlaufende Wulst oder Rippe, so dass das Dichtelement 5 fest auf dem Stößel 4 gehalten wird. Das Dichtelement 5 weist ein geschlossenes Ende 21 auf, das von dem Ventilschieber gegen den Dichtsitz 10 gedrückt wird, um diesen zu verschließen. An das geschlossene Ende 21 des Dichtelements 5 schließt sich ein fest eingespannter Faltenbalg 6 an, der axial deformierbar ist und so eine axiale Bewegung des Ventilschiebers erlaubt. Das Dichtelement 5 ist mit seinem von dem Dichtsitz 10 abgewandten Ende 22 fest im Ventilgehäuse dichtend eingeklemmt. Auf diese Weise ist der Ventilschieber fluiddicht von dem Fluidkanal 11 getrennt und kommt nicht mit Fluid in Kontakt, was insbesondere bei medizinischen Anwendungen von Bedeutung ist.

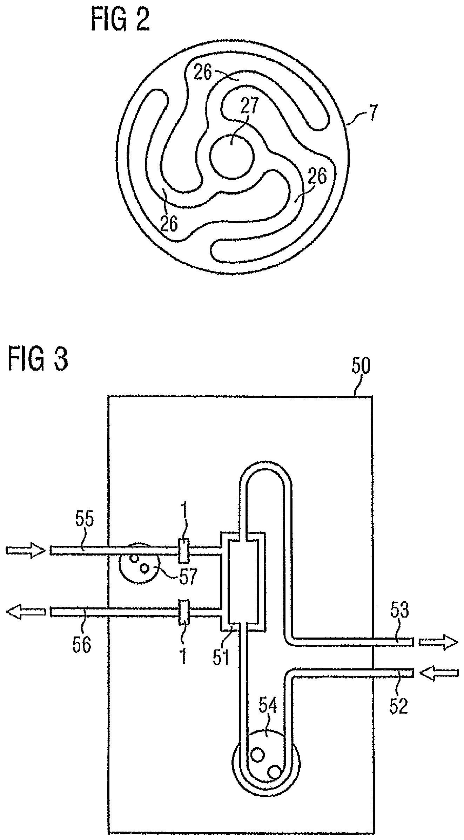

Auf der dem Dichtsitz 10 zugewandten Seite des Magnetankers 2 ist der Ventilschieber mittels eines Federelements 7 gelagert. Dadurch ist der Ventilschieber radial ausgerichtet und zugleich axial beweglich. Das Federelement 7 ist in Fig. 2 in der Draufsicht dargestellt. Es ist im entspannten Zustand eben ausgebildet und weist eine zentrale Öffnung 27 auf, in die der Ventilschieber eingeführt ist. Das Federelement 7 ist im stromlosen Zustand des Ventils 1, d.h. bei abgeschalteter Spule 12, in Richtung des Dichtsitzes 10 ausgelenkt, beispielsweise um 0,3 mm entlang der Verschiebeachse. Mittels geschwungener oder im Wesentlichen S-förmiger Arme 26 erfolgt eine radiale Zentrierung des Ventilschiebers. Der Stößel 4 ist in der zentralen Öffnung 27 des Federelements 7 aufgenommen. Das Federelement 7 ist in diesem Ausführungsbeispiel mit seinem inneren Rand zwischen dem Magnetanker 2 und einer Bördelung 18 des Stößels 4 eingeklemmt, so dass es relativ zu dem Ventilschieber ortsfest ist. Der äußere Rand des Federelements 7 ist seinerseits mit Gehäuseteilen des Ventils 1 ortsfest verbunden. Dadurch kommt es zu einer Auslenkung des Federelements 7 senkrecht zu seiner Ebene, wenn sich der Ventilschieber in axialer Richtung entlang der Verschiebeachse 20 bewegt. Das Federelement 7 wird dann um etwa den gleichen Betrag in die entgegengesetzte Richtung bezogen auf den ebenen Zustand des Federelements 7 ausgelenkt, um den es im stromlosen Zustand ausgelenkt ist, also beispielsweise um etwa 0,3 mm. Während der Bewegung des Ventilschiebers bleibt dieser durch das Federelement 7 in radialer Richtung ausgerichtet und zentriert.

An seinem dem Dichtsitz 10 gegenüberliegenden axialen Ende 8 weist der Ventilschieber einen Führungsstift 3 auf, der in axialer Richtung von dem Magnetanker 2 absteht. Der Führungsstift 3 ist mit einem Ende in den Magnetanker 2 eingepresst. Das andere Ende ist in einem Gleitlager 14 axial beweglich gelagert.

Der Führungsstift 3 kann beispielsweise aus Stahl oder Messing bestehen. Das Gleitlager 14 kann beispielsweise aus Kunststoff oder Stahl bestehen, wobei auch eine PTFE-Beschichtung zum Einsatz kommen kann. An dem Gleitlager 14 stützt sich die Feder 13 ab, die an ihrer gegenüberliegenden Seite in einer Bohrung in dem Magnetanker 2 sitzt. In diesem Ausführungsbeispiel drängt die Kraft der Feder 13 den Ventilschieber in die Schließposition.

Durch die spezielle Führung des Magnetankers 2, nämlich einerseits durch die radiale Ausrichtung mittels des Federelements 7 und andererseits durch die Führung mittels des Führungsstifts 3 in dem Gleitlager 14, ist es möglich, dass der Magnetanker 2 selbst im Wesentlichen berührungsfrei gelagert ist. Es kann auf ein zusätzliches Führungsrohr im Inneren der Spule 12 verzichtet werden. Stattdessen besteht ein Luftspalt 16 zwischen dem Magnetanker 2 und der Spule 12. Durch die berührungsfreie Lagerung des Magnetankers entsteht keine Reibung, die den Verschleiß und die notwendigen Magnetkräfte erhöhen würde. Durch den Verzicht auf ein Führungsrohr, welches nicht-magnetisch ausgebildet sein muss, können magnetische Verluste reduziert werden. Zudem kann durch den Wegfall eines Führungsrohrs der Luftspalt zwischen dem Magnetanker 2 und einer Flussleitscheibe 28 kleiner gestaltet werden, so dass geringere Verluste im Magnetkreis auftreten. Die axiale Bewegung des Führungsstifts 3 wird durch einen Anschlag 17 begrenzt, indem das freie axiale Ende 8 gegen den Anschlag 17 stößt. Der Anschlag 17 ist hier als geräuschdämpfende Gummischeibe ausgebildet und ist besonders einfach und kostengünstig herstellbar, beispielsweise als Formteil oder Stanzteil. Die Gummischeibe ist insbesondere im Vergleich zu O-Ringen, die alternativ als Dämpfungselement eingesetzt werden können, vorteilhaft, denn O-Ringe weisen beispielsweise den Nachteil auf, dass sie formgebunden sind und eine begrenzte Dämpfwirkung haben.

Es ist eine Kunststoffummantelung 15 vorgesehen, welche zumindest den Antriebsteil des Ventils 1 umgibt, d.h. denjenigen Teil des Ventils, welcher den Eisenkreis mit der Spule 12 beherbergt. Die Kunststoffummantelung 15 schützt das Ventil 1 vor Spritzwasser und Verschmutzung gemäß Schutzart IP54. Darüber hinaus bietet die Kunststoffummantelung 15 einen thermischen Berührschutz.

Diese thermische Isolierung ist vorgesehen, da sich das Ventil im Betrieb erwärmen kann. Durch die Kunststoffummantelung 15 führen elektrische Versorgungsanschlüsse 19. Die Kunststoffummantelung kann insbesondere im Niederdruckspritzguss hergestellt werden, was einfacher und kostengünstiger ist als eine Pulverbeschichtung des Ventilgehäuses oder der Schutz durch eine Vergussmasse.

In Fig. 3 ist schematisch ein Dialysegerät 50 mit dem Ventil 1 dargestellt. Weiterführende Details des Dialysegeräts 50 sind der Einfachheit halber nicht dargestellt. Das Dialysegerät 50 weist eine Eingangsleitung 52 zum Zuführen von zu reinigendem Blut von einem Patienten sowie eine Ausgangsleitung 53 zur Rückführung des gereinigten Bluts zum Patienten auf. Das Blut wird mittels einer Blutpumpe 54 durch den Kreislauf gepumpt. Ferner sind eine Eingangsleitung 55 zum Zuführen von frischem Dialysat und eine Ausgangsleitung 56 zum Abführen von verbrauchtem Dialysat vorgesehen. Das Dialysat wird mittels einer Dialysatpumpe 57 durch die Leitungen 55, 56 gepumpt. Die eigentliche Dialyse wird mittels einer Dialysemembran 51 durchgeführt, wobei das Blut des Patienten auf einer Seite und das Dialysat auf der anderen Seite der Membran 51 geführt werden. Im Kreislauf des Dialysats ist mindestens ein Ventil 1 vorgesehen, um den Fluss des Dialysats zu steuern. In einem Dialysegerät können beispielsweise bis zu 30 oder 50 derartige Ventile zum Einsatz kommen. In Fig. 3 ist beispielhaft und schematisch in den Leitungen 55, 56 jeweils ein Ventil 1 dargestellt.

## Patentansprüche

1. Elektromagnetisch betätigtes Ventil (1), umfassend:
- einen axial verschieblichen Ventilschieber (2, 3, 4), der eingerichtet ist, in einer Schließposition mit einem ersten axialen Ende (9) einen Dichtsitz (10) in einem Fluidkanal (11) des Ventils (1) zu verschließen,
- ein Dichtelement (5) mit einem Faltenbalg (6), das zwischen dem Dichtsitz (10) und dem ersten axialen Ende (9) des Ventilschiebers (2, 3, 4) angeordnet ist und den Ventilschieber (2, 3, 4) von dem Fluidkanal (11) fluiddicht trennt,
- einen axial beweglichen Magnetanker (2) als Teil des Ventilschiebers (2, 3, 4) und
- eine elektrisch bestrombare Spule (12),
wobei der Magnetanker (2) durch elektrische Bestromung der Spule (12) entgegen einer auf den Magnetanker (2) wirkenden mechanischen Belastung bewegbar ist, um den Ventilschieber (2, 3, 4) in die Schließposition oder aus der Schließposition zu bewegen, **dadurch gekennzeichnet, dass** der Ventilschieber (2, 3, 4) an zumindest einem seiner axialen Enden (8, 9) mittels mindestens eines Federelements (7) in radialer Richtung ausgerichtet und in axialer Richtung beweglich gelagert ist, wobei der Ventilschieber (2, 3, 4) an dem dem ersten axialen Ende (9) gegenüberliegenden zweiten axialen Ende (8) einen in axialer Richtung vom Magnetanker (2) abstehenden Führungsstift (3) aufweist, der eine Oberfläche mit einem gegenüber dem Außendurchmesser des Magnetankers (2) reduzierten Durchmesser besitzt, wobei der Ventilschieber (2,3,4) auf dieser Oberfläche des Führungsstifts (3) in axialer Richtung gleitend gelagert ist, und der Ventilschieber (2, 3, 4) auf der dem ersten axialen Ende (9) des Ventilschiebers (2, 3, 4) zugewandten Seite des Magnetankers (2) mittels des mindestens einen Federelements (7) in radialer Richtung ausgerichtet und in axialer Richtung beweglich gelagert ist.

2. Elektromagnetisch betätigtes Ventil (1), umfassend:
- einen axial verschieblichen Ventilschieber (2, 3, 4), der eingerichtet ist, in einer Schließposition mit einem ersten axialen Ende (9) einen Dichtsitz (10) in einem Fluidkanal (11) des Ventils (1) zu verschließen,
- ein Dichtelement (5) mit einem Faltenbalg (6), das zwischen dem Dichtsitz (10) und dem ersten axialen Ende (9) des Ventilschiebers (2, 3, 4) angeordnet ist und den Ventilschieber (2, 3, 4) von dem Fluidkanal (11) fluiddicht trennt,
- einen axial beweglichen Magnetanker (2) als Teil des Ventilschiebers (2, 3, 4) und
- eine elektrisch bestrombare Spule (12),
wobei der Magnetanker (2) durch elektrische Bestromung der Spule (12) entgegen einer auf den Magnetanker (2) wirkenden mechanischen Belastung bewegbar ist, um den Ventilschieber (2, 3, 4) in die Schließposition oder aus der Schließposition zu bewegen,
**dadurch gekennzeichnet, dass** der Ventilschieber (2, 3, 4) an zumindest einem seiner axialen Enden (8, 9) mittels mindestens eines Federelements (7) in radialer Richtung ausgerichtet und in axialer Richtung beweglich gelagert ist, wobei der Ventilschieber (2,3,4) auf der dem ersten axialen Ende (9) des Ventilschiebers (2, 3, 4) zugewandten Seite des Magnetankers (2) einen in axialer Richtung vom Magnetanker (2) abstehenden Führungsstift (3) aufweist, der eine Oberfläche mit einem gegenüber dem Außendurchmesser des Magnetankers (2) reduzierten Durchmesser besitzt, wobei der Ventilschieber (2, 3, 4) auf dieser Oberfläche des Führungsstifts (3) in axialer Richtung gleitend gelagert ist, und der Ventilschieber (2, 3, 4) auf dem dem ersten axialen Ende (9) gegenüberliegenden zweiten axialen Ende (8) mittels des mindestens einen Federelements (7) in radialer Richtung ausgerichtet und in axialer Richtung beweglich gelagert ist.

3. Ventil nach Anspruch 1 oder 2, wobei das Federelement (7) mit dem Ventilschieber (2, 3, 4) und einem Gehäuse des Ventils (1) ortsfest verbunden ist.

4. Ventil nach einem der Ansprüche 1 bis 3, wobei das Federelement (7) im entspannten Zustand eben ist und sich in einer Ebene senkrecht zur Verschiebeachse (20) des Ventilschiebers (2, 3, 4) erstreckt.

5. Ventil nach Anspruch 4, wobei das Federelement (7) im stromlosen Zustand des Ventils (1) in Richtung des Dichtsitzes (10) ausgelenkt und bei elektrischer Bestromung der Spule (12) in eine entgegengesetzte Richtung auslenkbar ist.

6. Ventil nach einem der Ansprüche 1 bis 5 wobei das Dichtelement (5) an einem Ende geschlossen ist und an dem ersten axialen Ende (9) des Ventilschiebers (2,3,4) derart angeordnet ist, dass das geschlossene Ende (21) des Dichtelements (5) in der Schließposition des Ventilschiebers (2, 3, 4) den Dichtsitz (10) fluiddicht verschließt.

7. Ventil nach einem der Ansprüche 1 bis 6, wobei das Federelement (7) ein ringförmiges Element mit geschwungenen oder im Wesentlichen s-förmigen Federbeinen ist, so dass der Ventilschieber mittels des Federelements zentriert ist.

8. Ventil nach einem der Ansprüche 1 bis 7, wobei der Ventilschieber (2, 3, 4) einen Stößel (4) umfasst, welcher mit dem Magnetanker (2) auf der dem ersten axialen Ende (9) des Ventilschiebers (2, 3, 4) zugewandten Seite des Magnetankers (2) verbunden ist und das erste axiale Ende (9) des Ventilschiebers (2, 3, 4) bildet.

9. Ventil nach Anspruch 8, wobei das Dichtelement (5) an dem ersten axialen Ende (9) des Ventilschiebers (2, 3, 4) auf dem Stößel (4) derart befestigt ist, dass es sich bei einer Verlagerung des Ventilschiebers (2,3,4) mitbewegt.

10. Ventil nach einem der Ansprüche 1 bis 9, umfassend einen Anschlag (17), welcher mit dem Führungsstift (3) so zusammenwirkt, dass er eine axiale Bewegung des Ventilschiebers (2, 3,4) begrenzt.

11. Ventil nach Anspruch 10, wobei der Anschlag (17) ein Dämpfungselement aus einem Gummimaterial umfasst.

12. Ventil nach einem der Ansprüche 1 bis 11, des Weiteren umfassend eine Kunststoffummantelung (15), welche zumindest einen Antriebsteil des Ventils (1) umgibt.

13. Vorrichtung (50) zum Bereitstellen eines extrakorporalen Kreislaufs zur Behandlung eines Patienten, insbesondere zur Dialyse, umfassend zumindest einen Fluidkanal (55, 56) und zumindest ein Ventil (1) nach einem der Ansprüche 1 bis 12 in dem Fluidkanal (55, 56) zur Steuerung eines Fluidflusses durch den Fluidkanal (55, 56).

## Claims

1. Electromagnetically actuated valve (1), comprising:
- an axially displaceable valve slide (2, 3, 4) which is designed, in a closure position, to close, with a first axial end (9), a sealing seat (10) in a fluid channel (11) of the valve (1),
- a sealing element (5) with a corrugated bellows (6), which is arranged between the sealing seat (10) and the first axial end (9) of the valve slide (2, 3, 4) and separates the valve slide (2, 3, 4) from the fluid channel (11) in a fluid-tight manner,
- an axially movable magnet armature (2) as part of the valve slide (2, 3, 4), and
- an electrically energizable coil (12),
wherein the magnet armature (2) is movable, by electrical energization of the coil (12), counter to a mechanical load acting on the magnet armature (2), so as to move the valve slide (2, 3, 4) into the closure position or from the closure position, **characterized in that** the valve slide (2, 3, 4), at at least one of its axial ends (8, 9), is oriented in a radial direction and mounted movably in an axial direction by means of at least one spring element (7), wherein the valve slide (2, 3, 4), at the second axial end (8) opposite the first axial end (9), has a guide pin (3) which protrudes in an axial direction away from the magnet armature (2) and which has a surface with a reduced diameter in comparison with the external diameter of the magnet armature (2), wherein the valve slide (2, 3, 4) is mounted slidably in an axial direction on this surface of the guide pin (3), and the valve slide (2, 3, 4), on the side of the magnet armature (2) facing toward the first axial end (9) of the valve slide (2, 3, 4), is oriented in a radial direction and mounted movably in an axial direction by means of the at least one spring element (7).

2. Electromagnetically actuated valve (1), comprising:
- an axially displaceable valve slide (2, 3, 4) which is designed, in a closure position, to close, with a first axial end (9), a sealing seat (10) in a fluid channel (11) of the valve (1),
- a sealing element (5) with a corrugated bellows (6), which is arranged between the sealing seat (10) and the first axial end (9) of the valve slide (2, 3, 4) and separates the valve slide (2, 3, 4) from the fluid channel (11) in a fluid-tight manner,
- an axially movable magnet armature (2) as part of the valve slide (2, 3, 4), and
- an electrically energizable coil (12),
wherein the magnet armature (2) is movable, by electrical energization of the coil (12), counter to a mechanical load acting on the magnet armature (2), so as to move the valve slide (2, 3, 4) into the closure position or from the closure position, **characterized in that** the valve slide (2, 3, 4), at at least one of its axial ends (8, 9), is oriented in a radial direction and mounted movably in an axial direction by means of at least one spring element (7), wherein the valve slide (2, 3, 4), on the side of the magnet armature (2) facing toward the first axial end (9) of the valve slide (2, 3, 4), has a guide pin (3) which protrudes in an axial direction from the magnet armature (2) and which has a surface with a reduced diameter in comparison with the external diameter of the magnet armature (2), wherein the valve slide (2, 3, 4) is mounted slidably in an axial direction on this surface of the guide pin (3), and the valve slide (2, 3, 4), on the second axial end (8) opposite the first axial end (9), is oriented in a radial direction and mounted movably in an axial direction by means of the at least one spring element (7).

3. Valve according to Claim 1 or 2, wherein the spring element (7) is connected in a fixed position to the valve slide (2, 3, 4) and to a housing of the valve (1) .

4. Valve according to one of Claims 1 to 3, wherein the spring element (7) is planar in the relaxed state and extends in a plane perpendicular to the axis of displacement (20) of the valve slide (2, 3, 4).

5. Valve according to Claim 4, wherein the spring element (7) is deflected in the direction of the sealing seat (10) in the currentless state of the valve (1) and can be deflected in an opposite direction when the coil (12) is electrically energized.

6. Valve according to one of Claims 1 to 5, wherein the sealing element (5) is closed at one end and is arranged at the first axial end (9) of the valve slide (2, 3, 4) in such a way that the closed end (21) of the sealing element (5) closes the sealing seat (10) in a fluid-tight manner in the closure position of the valve slide (2, 3, 4).

7. Valve according to one of Claims 1 to 6, wherein the spring element (7) is an annular element with curved or substantially S-shaped spring legs, such that the valve slide is centred by means of the spring element.

8. Valve according to one of Claims 1 to 7, wherein the valve slide (2, 3, 4) comprises a ram (4) which is connected to the magnet armature (2), on the side of the magnet armature (2) facing toward the first axial end (9) of the valve slide (2, 3, 4), and which forms the first axial end (9) of the valve slide (2, 3, 4).

9. Valve according to Claim 8, wherein the sealing element (5), at the first axial end (9) of the valve slide (2, 3, 4), is secured to the ram (4) in such a way that it also moves upon displacement of the valve slide (2, 3, 4).

10. Valve according to one of Claims 1 to 9, comprising a stop (17) which interacts with the guide pin (3) such that it limits an axial movement of the valve slide (2, 3, 4).

11. Valve according to Claim 10, wherein the stop (17) comprises a damping element made of a rubber material.

12. Valve according to one of Claims 1 to 11, further comprising a plastic sheathing (15), which surrounds at least one drive part of the valve (1).

13. Device (50) for making available an extracorporeal circulation for treatment of a patient, in particular for dialysis, comprising at least one fluid channel (55, 56) and at least one valve (1) according to one of Claims 1 to 12 in the fluid channel (55, 56) in order to control a flow of fluid through the fluid channel (55, 56).

## Revendications

1. Soupape à commande électromagnétique (1), comprenant :
- un tiroir de soupape (2, 3, 4) axialement coulissant qui est aménagé, dans une position de fermeture, pour fermer par une première extrémité axiale (9) un siège d'étanchéité (10) dans un conduit de fluide (11) de la soupape (1),
- un élément d'étanchéité (5) doté d'un soufflet (6) qui est disposé entre le siège d'étanchéité (10) et la première extrémité axiale (9) du tiroir de soupape (2, 3, 4) et sépare de manière étanche au fluide le tiroir de soupape (2, 3, 4) du conduit de fluide (11),
- une armature (2) axialement mobile faisant partie du tiroir de soupape (2, 3, 4), et
- une bobine (12) pouvant être alimentée en courant,
l'armature (2) pouvant être déplacée par une alimentation électrique de la bobine (12) à l'opposé d'une charge mécanique agissant sur l'armature (2) afin de déplacer le tiroir de soupape (2, 3, 4) dans la position de fermeture ou hors de la position de fermeture,
**caractérisée en ce que** le tiroir de soupape (2, 3, 4) est orienté dans la direction radiale à au moins l'une de ses extrémités axiales (8, 9) au moyen d'au moins un élément faisant ressort (7) et est monté mobile dans la direction axiale, le tiroir de soupape (2, 3, 4) présentant à la deuxième extrémité axiale (8), opposée à la première extrémité axiale (9), une goupille de guidage (3) faisant saillie à partir de l'armature (2) dans la direction axiale et possédant une surface ayant un diamètre réduit par rapport au diamètre extérieur de l'armature (2), le tiroir de soupape (2, 3, 4) étant monté coulissant dans la direction axiale sur cette surface de la goupille de guidage (3), et le tiroir de soupape (2, 3, 4) étant orienté dans la direction radiale sur le côté, tourné vers la première extrémité axiale (9) du tiroir de soupape (2, 3, 4), de l'armature (2) au moyen du au moins un élément faisant ressort (7), et monté mobile dans la direction axiale.

2. Soupape à commande électromagnétique (1), comprenant :
- un tiroir de soupape (2, 3, 4) axialement coulissant qui, dans une position de fermeture, est aménagé pour fermer par une première extrémité axiale (9) un siège d'étanchéité (10) dans un conduit de fluide (11) de la soupape (1),
- un élément d'étanchéité (5) doté d'un soufflet (6) qui est disposé entre le siège d'étanchéité (10) et la première extrémité axiale (9) du tiroir de soupape (2, 3, 4) et sépare de manière étanche au fluide le tiroir de soupape (2, 3, 4) du conduit de fluide (11),
- une armature (2) axialement mobile faisant partie du tiroir de soupape (2, 3, 4), et
- une bobine (12) pouvant être alimentée en courant,
l'armature (2) pouvant être déplacée par une alimentation électrique de la bobine (12) à l'opposé d'une charge mécanique agissant sur l'armature (2) afin de déplacer le tiroir de soupape (2, 3, 4) dans la position de fermeture ou hors de la position de fermeture,
**caractérisée en ce que** le tiroir de soupape (2, 3, 4) est orienté dans la direction radiale à au moins l'une de ses extrémités axiales (8, 9) au moyen d'au moins un élément faisant ressort (7), et est monté mobile dans la direction axiale, le tiroir de soupape (2, 3, 4) présentant sur le côté, tourné vers la première extrémité axiale (9) du tiroir de soupape (2, 3, 4), de l'armature (2) une goupille de guidage (3) faisant saillie à partir de l'armature (2) dans la direction axiale et possédant une surface ayant un diamètre réduit par rapport au diamètre extérieur de l'armature (2), le tiroir de soupape (2, 3, 4) étant monté coulissant dans la direction axiale sur cette surface de la goupille de guidage (3), et le tiroir de soupape (2, 3, 4) étant orienté dans la direction radiale à la deuxième extrémité axiale (8) opposée à la première extrémité axiale (9) au moyen du au moins un élément faisant ressort (7), et étant monté mobile dans la direction axiale.

3. Soupape selon la revendication 1 ou 2, dans laquelle l'élément faisant ressort (7) est relié de manière stationnaire au tiroir de soupape (2, 3, 4) et à une cage de la soupape (1).

4. Soupape selon l'une quelconque des revendications 1 à 3, dans laquelle l'élément faisant ressort (7) est plat à l'état détendu et s'étend dans un plan perpendiculaire à l'axe de décalage (20) du tiroir de soupape (2, 3, 4).

5. Soupape selon la revendication 4, dans laquelle l'élément faisant ressort (7), à l'état sans tension de la soupape (1), est dévié en direction du siège d'étanchéité (10), et en cas d'alimentation électrique de la bobine (12), peut être dévié dans une direction opposée.

6. Soupape selon l'une quelconque des revendications 1 à 5, dans laquelle l'élément d'étanchéité (5) est fermé à une extrémité et est disposé à la première extrémité axiale (9) du tiroir de soupape (2, 3, 4) de telle sorte que l'extrémité fermée (21) de l'élément d'étanchéité (5), dans la position de fermeture du tiroir de soupape (2, 3, 4), ferme de manière étanche au fluide le siège d'étanchéité (10).

7. Soupape selon l'une quelconque des revendications 1 à 6, dans laquelle l'élément faisant ressort (7) est un élément annulaire doté de branches élastiques courbes ou substantiellement en forme de S de sorte que le tiroir de soupape est centré au moyen de l'élément faisant ressort.

8. Soupape selon l'une quelconque des revendications 1 à 7, dans laquelle le tiroir de soupape (2, 3, 4) comprend un poussoir (4) qui est relié à l'armature (2) sur le côté, tourné vers la première extrémité axiale (9) du tiroir de soupape (2, 3, 4), de l'armature (2) et constitue la première extrémité axiale (9) du tiroir de soupape (2, 3, 4).

9. Soupape selon la revendication 8, dans laquelle l'élément d'étanchéité (5) est fixée à la première extrémité axiale (9) du tiroir de soupape (2, 3, 4) sur le poussoir (4) de telle sorte qu'il se déplace conjointement en cas de décalage du tiroir de soupape (2, 3, 4).

10. Soupape selon l'une quelconque des revendications 1 à 9, comprenant une butée (17) qui coopère avec la goupille de guidage (3) de telle sorte qu'elle limite un mouvement axial du tiroir de soupape (2, 3, 4).

11. Soupape selon la revendication 10, dans laquelle la butée (17) comprend un élément d'amortissement en matériau caoutchouteux.

12. Soupape selon l'une quelconque des revendications 1 à 11, comprenant en outre une gaine plastique (15) qui entoure au moins une partie d'entraînement de la soupape (1).

13. Dispositif (50) permettant de fournir un circuit extracorporel destiné au traitement d'un patient, en particulier pour la dialyse, comprenant au moins un conduit de fluide (55, 56) et au moins une soupape (1) selon l'une quelconque des revendications 1 à 12 dans le conduit de fluide (55, 56) pour commander un flux de fluide à travers le conduit de fluide (55, 56).
